(19) Europäisches Patentamt
European Patent Office
Office européen des brevets

(11) EP 1 230 914 A1

(12) **DEMANDE DE BREVET EUROPEEN**

(43) Date de publication:
**14.08.2002 Bulletin 2002/33**

(51) Int Cl.[7]: **A61K 7/48**, A61K 7/40

(21) Numéro de dépôt: **02290093.0**

(22) Date de dépôt: **15.01.2002**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **07.02.2001 FR 0101645**

(71) Demandeur: **L'OREAL**
**75008 Paris (FR)**

(72) Inventeurs:
- **Labrousse, Emmanuelle**
  **91430 Igny (FR)**
- **Cotovio, José**
  **77230 Dammartin en Goele (FR)**

(74) Mandataire: **Renard, Emmanuelle**
**L'OREAL-DPI**
**6 rue Bertrand Sincholle**
**92585 Clichy Cedex (FR)**

(54) **Composition topique anti-pollution**

(57) La présente demande se rapporte à l'utilisation en application topique, d'un amidon, comme agent anti-pollution, notamment comme agent cosmétique anti-pollution.

La demande se rapporte aussi à un procédé de traitement cosmétique en vue de protéger l'organisme contre les effets de la pollution, consistant à appliquer sur la matière kératinique, une composition contenant dans un milieu physiologiquement acceptable, une quantité efficace d'au moins un amidon.

La demande se rapporte également à une composition pour application topique contenant dans un milieu physiologiquement acceptable au moins un amidon et au moins une gomme de silicone, et à ses utilisations dans les domaines cosmétique et dermatologique.



Printed by Jouve, 75001 PARIS (FR)

## Description

La présente demande se rapporte à l'utilisation en application topique, d'amidon comme agent anti-pollution, et à un procédé de traitement cosmétique en vue de protéger l'organisme contre les effets de la pollution, consistant à appliquer sur la matière kératinique, une composition contenant dans un milieu physiologiquement acceptable, une quantité efficace d'amidon.

La demande se rapporte également à une composition anti-pollution pour application topique contenant dans un milieu physiologiquement acceptable au moins un amidon et au moins une gomme de silicone, et à ses utilisations dans les domaines cosmétique et dermatologique.

Les milieux urbains sont régulièrement soumis à des pics de pollution. L'individu dans son environnement quotidien et particulièrement en zone urbaine, peut être soumis à de multiples agressions au niveau des matières kératiniques, et notamment de la peau, du cuir chevelu et des cheveux, par différents polluants aériens. Les polluants atmosphériques qui sont représentés largement par les produits primaires et secondaires de la combustion représentent une source importante du stress oxydatif environnemental. Différents types de produits chimiques, xénobiotiques et particules, composent la pollution urbaine. Les grandes catégories de polluants pouvant exercer des effets délétères sur la peau et le cheveu sont les suivants : les gaz, les métaux lourds, les hydrocarbures aromatiques polycycliques (HAP) et les éléments particulaires qui sont des résidus de combustion sur lesquelles sont adsorbés de très nombreux composés organiques et minéraux.

Ce sont les tissus les plus externes qui sont initialement et directement exposés aux toxiques de l'environnement. La peau est directement et fréquemment exposée à l'environnement pro-oxydant et elle est particulièrement sensible à l'action du stress oxydatif ; sa couche la plus externe sert de barrière vis-à-vis des dommages oxydatifs pouvant se produire. Dans la plupart des circonstances, l'oxydant est généralement neutralisé après réaction avec les matières kératiniques, mais les produits de réaction formés peuvent être responsables d'atteintes cellulaires et tissulaires. Le *stratum corneum*, la barrière de la peau, est le site de contact entre air et tissu cutané, et la structure biphasique lipides/protéines est un déterminant crucial de cette fonction de barrière de la peau. Ces éléments peuvent réagir avec les oxydants et être altérés, ce qui favorisera les phénomènes de desquamation.

Parmi les polluants pouvant exercer des effets délétères sur les matières kératiniques, les gaz toxiques tels que l'ozone, le monoxyde de carbone, les oxydes d'azote ou les oxydes de soufre, sont des constituants majeurs des polluants. Il a été constaté que ces gaz toxiques favorisent la desquamation des matières kératiniques, "fatigue" lesdites matières kératiniques, c'est-à-dire les rendent sales et ternes. De même, il a été constaté une asphyxie cellulaire des dites matières kératiniques.

On sait par ailleurs que les métaux lourds (plomb, cadmium, mercure) sont des polluants atmosphériques dont les émissions ont notablement augmenté, notamment en milieu urbain ou industriel. Bien que la majorité des effets de ces métaux sont décrits dans d'autres tissus (poumons, reins, cerveau...), il a été montré que certains métaux peuvent pénétrer dans la peau et s'y accumuler (A.B.G. Landsdown. Critical Reviews in Toxicology, 1995, vol. 25, p. 397-462). A fortes concentrations, les métaux lourds peuvent induire des mécanismes d'oxydation des lipides membranaires, une cytotoxicité directe capable d'aboutir à une nécrose cellulaire, une alkylation des nucléophiles cellulaires via des mécanismes pouvant être à l'origine de phénomènes de sensibilisation ou de cancérogénèse (S.J. Stochs and D. Bagchi, Free Radical Biology and Medicine.1995, vol. 18, p. 321-336 ; M. E. Figueiredo-Pereira et al., The Journal of Biological Chemistry, 1998, vol. 21, p. 12703-12709.; N. L. Acan et coll., 1995, Biochemical and Molecular Medicine, vol. 54, p. 33-37).

Outre certains effets toxiques qui leur sont propres, les métaux lourds ont la propriété de diminuer l'activité des moyens de défense cellulaire contre les radicaux libres [voir par exemple R.S. Dwivedi, J. Toxicol. Cut. & Ocular Toxical. 6(3), 183-191 (1987)]. Ainsi, les métaux lourds aggravent les effets toxiques des polluants gazeux en diminuant l'efficacité des moyens naturels de défense, et provoquent une accélération du phénomène de vieillissement cellulaire. Ceci est vrai en particulier pour les matières kératiniques et notamment la peau, le cuir chevelu et les cheveux qui sont en contact direct et permanent avec le milieu extérieur.

Une autre catégorie majeure de polluants est constituée par des résidus de combustion sous forme de particules sur lesquelles sont adsorbés de très nombreux composés organiques, et en particulier des hydrocarbures aromatiques polycycliques (HAP). Ces HAP adsorbés à la surface des particules et des poussières véhiculées par l'air urbain, peuvent pénétrer le tissu cutané et y être stockés et /ou biotransformés. Leur métabolisme hépatique, bien décrit dans la littérature, conduit à des formations de métabolites monohydroxylés (voie de détoxification), d'époxydes et de diols époxydes (voie toxifiante). On peut observer des phénomènes similaires au niveau cutané. Ces composés sont connus pour avoir des effets carcinogènes et immunogènes au niveau cutané.

Ainsi, les effets nocifs de la pollution sur les matières kératiniques affectent la respiration cellulaire de ces matières kératiniques et se traduisent par un vieillissement accéléré de la peau, avec un teint terne et la formation précoce de rides ou ridules, et aussi par une diminution de la vigueur des cheveux qui prennent aussi un aspect terne. En outre, du fait de la pollution, peau et cheveux se salissent plus rapidement. De plus, la pollution peut provoquer

des irritations et des phénomènes d'allergie et d'inflammation sur la peau.

Pour lutter contre ces effets des polluants, divers agents anti-pollution ont été décrits. Ainsi le document EP-A-557 042 décrit l'utilisation des métallothionéines pour protéger les tissus contre les métaux lourds. Par ailleurs, le document EP-A-577 718 décrit l'utilisation des sphingolipides pour protéger la peau et les cheveux de la pollution atmosphérique.

Avec l'augmentation de la pollution, il subsiste le besoin de trouver d'autres agents permettant de lutter efficacement contre l'effet néfaste des polluants sur les matières kératiniques et empêcher l'adhérence de ces polluants sur les matières kératiniques, notamment pour éviter la dégradation de la respiration cellulaire, la desquamation et le vieillissement accéléré des matières kératiniques et notamment de la peau, ainsi que lutter contre le teint terne et la formation précoce de rides ou ridules sur la peau, pour éviter que les cheveux aient un aspect terne et se salissent, et pour éviter l'irritation de la peau ainsi que les phénomènes d'allergie cutanée et d'inflammation de la peau.

La demanderesse a maintenant trouvé, de façon tout à fait surprenante, que l'utilisation d'un ou plusieurs amidons, permettait de protéger les matières kératiniques des effets des polluants et notamment des polluants particulaires, et qu'en outre, l'association d'un amidon avec une gomme de silicone permettait d'augmenter l'effet anti-pollution de l'amidon.

Certes, il est connu d'utiliser l'amidon dans des compositions à application topique et notamment cosmétiques, destinées à être appliquées sur la peau, par exemple comme charge (voir par exemple EP-A-925 777 et EP-A-745 379). Toutefois, aucun document ne décrit que ces composés puissent avoir des propriétés de protection des matières kératiniques contre la pollution.

Ainsi, l'invention a pour objet l'utilisation cosmétique d'au moins un amidon, comme agent cosmétique anti-pollution, dans une composition pour application topique sur les matières kératiniques.

L'invention a aussi pour objet l'utilisation d'au moins un amidon, pour la préparation d'une composition à application topique destinée à protéger les matières kératiniques contre les effets nocifs de la pollution, notamment de la pollution particulaire, tels que l'inflammation de la peau et les problèmes d'allergie cutanée.

On entend par « agent anti-pollution » un agent qui protège la peau et les matières kératiniques de façon à prévenir, atténuer et/ou supprimer les effets délétères d'agents polluants (ex. HAP, métaux lourds, etc...) notamment ceux adsorbés sur les particules.

Dans le cadre de la présente invention, on entend par « matière kératinique » la peau, le cuir chevelu, les cheveux, les cils, les sourcils, les ongles et les muqueuses.

On entend ici par « application topique » une application externe sur les matières kératiniques, que sont notamment la peau, le cuir chevelu, les cils, les sourcils, les ongles et les muqueuses, et de manière générale tout tissu cutané.

La composition utilisée selon l'invention est destinée à une application topique et contient donc un milieu physiologiquement acceptable, c'est-à-dire compatible avec les tissus cutanés tels que la peau, le cuir chevelu, les cils, les sourcils, les ongles et les muqueuses. Ainsi, la composition peut être appliquée sur tout le corps humain.

Le ou les amidons utilisés comme composés anti-pollution selon l'invention sont avantageusement présents en une quantité suffisante. On entend ici par « quantité suffisante » (ou quantité efficace), une quantité telle que la protection contre les polluants soit assurée. Cette quantité peut aller par exemple de 0,05 à 15 % en poids, de préférence de 0,1 à 10 % en poids et mieux de 0,5 à 7 % en poids de matière active de composé(s) anti-pollution par rapport au poids total de la composition. Cette quantité varie selon le ou les composés utilisés et le milieu de la composition.

L'amidon est un produit naturel bien connu de l'homme du métier. Il consiste en un polymère ou un mélange de polymères, linéaires ou branchés, constitués d'unités d'$\alpha$-D-glucopyranosyle. L'amidon est décrit en particulier dans "KIRK-OTHMER ENCYCLOPEDIA OF CHEMICAL TECHNOLOGY, 3$^{ème}$ édition, volume 21, p.492-507, Wiley Interscience, 1983".

Les amidons et ses dérivés utilisables dans la présente invention sont plus particulièrement des macromolécules sous forme de polymères constitués de motifs élémentaires qui sont des unités anhydroglucose. Le nombre de ces motifs et leur assemblage permettent de distinguer l'amylose (polymère linéaire) et l'amylopectine (polymère ramifié). Les proportions relatives d'amylose et d'amylopectine, ainsi que leur degré de polymérisation, varient en fonction de l'origine botanique des amidons.

Les amidons utilisés dans la présente invention peuvent avoir comme origine botanique les céréales ou encore les tubercules. Ainsi, les amidons sont par exemple choisis parmi les amidons de maïs, de riz, de manioc, de pomme de terre, de blé, de sorgho, de pois, natifs (c'est-à-dire non modifiés) ou modifiés, et leurs mélanges. Parmi les amidons modifiés, on peut citer les amidons précuits, les amidons hydrolysés, les amidons réticulés, par exemple par un dérivé de méthylolurée ou par l'anhydride octénylsuccinique ou encore par l'épichlorhydrine, les amidons estérifiés, les amidons éthérifiés, les amidons oxydés, les amidons raffinés, les amidons grillés en présence d'acide, ou encore les amidons greffés, par exemple par des polyacrylates de sodium, les amidons enrobés, par exemple par des aminoacides, et/ou leurs mélanges.

Les amidons se présentent généralement sous la forme d'une poudre blanche, insoluble dans l'eau froide,

dont la taille des particules élémentaires va par exemple de 3 à 100 microns. Cette poudre forme un gel lorsqu'elle est chauffée.

Parmi les amidons et dérivés convenant particulièrement à l'invention, on peut citer :

- l'amidon de maïs (amidon non modifié) tel que le produit commercialisé sous la dénomination « Amidon de mais B » par la société ROQUETTE FRERES et le produit commercialisé sous la dénomination « Amidon de mais » par la société NATIONAL STARCH ;
- l'amidon modifié (amylopectine/amylose réticulée par épichlorhydrine) vendu sous la dénomination commerciale « Amidon de riz insoluble non mucilagineux » par la société REMY,
- l'amidon de maïs cireux (amylopectine essentiellement) natif, vendu sous la dénomination commerciale « WAXY MAIS » par la société BLATTMANN,
- l'amidon de blé modifié et précuit, vendu sous la dénomination commerciale « MIDSOL KRISP » par la société MIDWEST GRAIN PRODUCTS,
- l'amidon de blé modifié et raffiné, vendu sous la dénomination commerciale « MIDSOL ADHERE » par la société MIDWEST GRAIN PRODUCTS,
- la poudre d'amidon de blé modifiée, vendue sous la dénomination commerciale « MIDSOL 35 » par la société MIDWEST GRAIN PRODUCTS,
- l'amidon de pomme de terre modifié, vendu sous la dénomination commerciale « PERFECTAGEL MPT » par la société AVEBE.

On peut utiliser un mélange de ces amidons.

Dans une forme particulièrement avantageuse de l'invention, l'amidon utilisé est un amidon non modifié et de préférence l'amidon de maïs.

On peut de manière avantageuse ajouter à l'amidon ou aux amidons utilisés selon l'invention, une ou plusieurs gommes de silicone.

Dans le cadre de la présente invention, on désigne sous le terme "gomme de silicone" des polydiméthylsiloxanes linéaires non réticulés qui peuvent être hydroxylés ou phénylés et qui ont la consistance d'une huile épaisse ou d'un solide transparent par opposition avec les alkyl- ou alkoxy-diméthicones qui, lorsqu'ils sont solides, présentent un aspect opaque cireux, mais peuvent également avoir l'aspect d'une huile limpide lorsque leur point de fusion est inférieur à la température ambiante.

La gomme de silicone utilisée selon l'invention peut être choisie notamment parmi des polydiorganosiloxanes de masse moléculaire allant de 100 000 à 2 000 000, et préférentiellement de 100 000 à 1 500 000. Ces gommes de silicone présentent préférentiellement une viscosité égale ou supérieure à 200.000 cSt (0,2 $m^2/s$) et préférentiellement supérieure à 300.000 cSt (0,3 $m^2/s$), viscosité mesurée au viscosimètre BROOKFIELD à 25°C.

On utilise plus particulièrement la gomme de silicone choisie parmi les composés de formule (I) suivante :

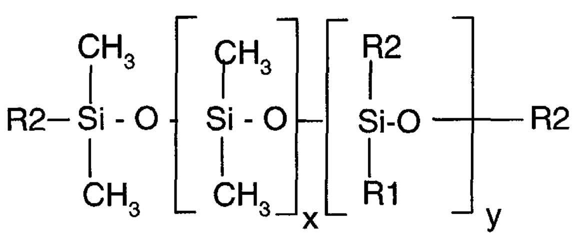

(I)

dans laquelle :

R1 représente $-CH_3$, -OH, $-C_6H_5$ ou $-OSi(CH_3)_3$,
R2 représente $-CH_3$, -OH ou $-C_6H_5$,
x = 0 ou un nombre entier et
y est un nombre entier,
y ou x + y étant des nombres entiers tels que la masse moléculaire moyenne en poids soit supérieure à 100 000, et de préférence aille de 100 000 à 1 500 000.

Les gommes de silicone préférées de l'invention sont choisies parmi les diméthicones (polydiméthylsiloxanes) et plus particulièrement les diméthiconols (polydiméthylsiloxanes à terminaison hydroxyle).

On peut utiliser une ou plusieurs gommes de silicone, et, la ou les gommes de silicone peuvent se présenter

telles quelles (à 100 % de matière active) ou en mélange avec un solvant, notamment un solvant choisi parmi les silicones volatiles, les huiles polydiméthylsiloxanes, les huiles polyphénylméthylsiloxanes, les isoparaffines, le chlorure de méthylène, le pentane, le dodécane, le tridécane, le tétradécane ou leurs mélanges.

Les gommes de silicone sont généralement commercialisées par les fournisseurs en mélange avec un polydiméthylsiloxane linéaire ou cyclique, de faible poids moléculaire, volatil ou non volatil, dans une proportion de 5 à 20 % en poids de matière active, et de préférence de 10 à 15 % en poids de matière active par rapport au poids total du mélange.

Comme gommes de silicone, on peut citer à titre d'exemple, la diméthicone à 96% dans du cyclométhicone, vendue par la Société RHONE POULENC sous la dénomination de Mirasil DM-500000® ; la diméthicone vendue par la société WACKER sous la dénomination AK 300000®, et les diméthiconols en mélange avec un diméthicone ou une cyclométhicone, vendus sous les dénominations de Q2-1403®, Q2-1401®, Q2-1503® et DC2-9085® par la Société DOW CORNING.

L'invention se rapporte aussi à une composition à application topique contenant dans un milieu physiologiquement acceptable, au moins un amidon non modifié choisi parmi les amidons de maïs, de riz, de manioc, de pomme de terre, de blé, de sorgho et de pois, et au moins une gomme de silicone.

Dans la composition selon l'invention, la quantité d'amidon va de préférence de 0,05 à 15 % en poids (de matière active), mieux de 0,1 à 10 % en poids et encore mieux de 0,5 à 7 % en poids par rapport au poids total de la composition, et la quantité de gomme de silicone va de préférence de 0,01 à 10 % en poids (de matière active), mieux de 0,05 à 5 % en poids et encore mieux de 0,1 à 3 % en poids par rapport au poids total de la composition.

Les compositions à application topique, et notamment cosmétiques, utilisées selon l'invention contiennent un milieu physiologiquement acceptable. Ce milieu physiologiquement acceptable peut être plus particulièrement constitué d'eau et éventuellement d'un solvant organique physiologiquement acceptable choisi par exemple parmi les alcools inférieurs comportant de 1 à 8 atomes de carbone et en particulier 1 à 6 atomes de carbone, comme l'éthanol, l'isopropanol, le propanol, le butanol ; les polyéthylène glycols ayant de 6 à 80 oxydes d'éthylène ; les polyols comme le propylène glycol, l'isoprène glycol, le butylène glycol, la glycérine, le sorbitol. Ce peut être aussi un milieu anhydre, notamment un milieu huileux contenant des huiles et/ou matières grasses autres que les huiles.

Quand le milieu physiologiquement acceptable est un milieu aqueux, il a un pH compatible avec la peau, allant de préférence de 3 à 8 et mieux de 4 à 7.

Quand la composition comporte un milieu aqueux ou hydroalcoolique, il est possible d'ajouter une phase grasse (ou huileuse) dans ce milieu, afin que les compositions de l'invention soient plus douces et plus nourrissantes.

Ainsi, les compositions selon l'invention contenant les agents anti-pollution tels que définis ci-dessus peuvent se présenter sous toutes les formes galéniques classiquement utilisées pour une application topique et notamment sous forme de solutions aqueuses, hydroalcooliques ou huileuses, d'émulsions huile-dans-eau (H/E) ou eau-dans-huile (E/H) ou multiple (triple : E/H/E ou H/E/H), de gels aqueux ou huileux, de produits anhydres liquides, pâteux ou solides, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique. Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Quand la composition utilisée selon l'invention comporte une phase huileuse, celle-ci contient de préférence au moins une huile. Elle peut contenir en outre d'autres corps gras.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules $R^1COOR^2$ et $R^1OR^2$ dans laquelle $R^1$ représente le reste d'un acide gras comportant de 8 à 29 atomes de carbone, et $R^2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les

heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle et le tétraoctanoate de pentaérythrityle ;

- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition contenant les composés anti-pollution est une émulsion eau-dans-huile (E/H) ou huile-dans-eau (H/E), et plus particulièrement une émulsion H/E. La proportion de la phase huileuse de l'émulsion peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90$^{R}$ par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de pEG-40 commercialisé sous la dénomination ARLACEL 165 par la société Uniqema.

De façon connue, la composition cosmétique ou dermatologique de l'invention peut contenir également des adjuvants habituels dans le domaine cosmétique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur, les matières colorantes, les sels. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

Comme gélifiants hydrophiles, on peut citer par exemple les polymères carboxyvinyliques comme les produits commercialisés sous les dénominations Carbopol (nom CTFA :carbomer) par la société Goodrich, les copolymères acryliques tels que les copolymères d'acrylates/alkylacrylates, les polyacrylamides, les polysaccharides tels que les dérivés de cellulose et notamment l'hydroxyéthylcellulose, les gommes naturelles et les argiles. Comme gélifiants lipophiles, on peut citer les argiles modifiées comme les bentones, les sels métalliques d'acides gras, la silice hydrophobe et les polyéthylènes.

Selon un mode préféré de réalisation de l'invention, la composition utilisée selon l'invention contient au moins un filtre U.V. (ou filtre solaire) qui peut être un filtre chimique ou un filtre physique ou un mélange de tels filtres.

Des exemples de filtres U.V. convenant particulièrement bien à une utilisation dans la présente invention sont :

- le butylméthoxydibenzoylméthane vendu notamment par la société Hoffmann-Laroche sous la dénomination Parsol 1789,
- l'octocrylène vendu notamment par la société BASF sous la dénomination Uvinul N539,
- l'octylsalicylate vendu notament par la société Haarman-Reimer sous la dénomination Neo Heliopan OS,
- l'octylméthoxycinnamate vendu notamment par la société Hoffmann-Laroche sous la dénomination Parsol MCX,
- l'acide phénylbenzimidazole sulfonique vendu notamment par la société Merck sous la dénomination Eusolex 232,
- les oxybenzones telles que les benzophénones-3, -4 ou -5,
- les silicones benzotriazoles et en particulier le drométrizole trisiloxane,
- l'acide téréphtalylidène di-camphre sulfonique, et
- les oxydes de titane ou de zinc, sous forme de micro- ou nanoparticules (nanopigments) éventuellement enrobées.

On utilise de préférence comme filtre dans la composition de l'invention l'octyl méthoxycinnamate (Parsol MCX de la société Hoffmann-Laroche), l'acide téréphtalylidène di-camphre sulfonique (MEXORYL SX de la société Chimex), le benzophénone-3 (Uvinul M40 de la société BASF), l'acide phénylbenzimidazole sulfonique (Eusolex 232 de la société Merck) et leurs mélanges.

La quantité de filtres dépend de l'utilisation finale souhaitée. Elle peut aller par exemple de 0,5 à 20 % en poids, de préférence de 2 à 15 % en poids et mieux de 2 à 10 % en poids par rapport au poids total de la composition.

Les compositions utilisées selon l'invention peuvent notamment constituer un produit de soin et/ou de maquillage des matières kératiniques, et notamment de la peau. Elles peuvent être utilisées notamment pour protéger l'organisme, en particulier les matières kératiniques, contre les effets de la pollution, notamment pour améliorer la respiration cellulaire et/ou diminuer la desquamation et/ou pour éviter de ternir ou de salir les matières kératiniques, et notamment la peau.

Ainsi, un autre objet de l'invention consiste en un procédé de traitement cosmétique en vue de protéger une matière kératinique (peau, cheveux ou autres) contre les effets de la pollution, consistant à appliquer sur la matière kératinique une composition contenant une quantité efficace d'au moins un amidon.

L'invention a aussi pour objet un procédé de traitement cosmétique d'une matière kératinique en vue d'améliorer sa respiration cellulaire et/ou de diminuer sa desquamation et/ou pour éviter de la ternir et/ou de la salir, consistant à appliquer sur la matière kératinique, une composition contenant dans un milieu physiologiquement acceptable, une quantité efficace d'au moins un amidon.

Les exemples qui suivent servent à illustrer l'invention sans toutefois présenter un caractère limitatif. Les noms sont, selon le cas, en noms chimiques ou noms CTFA (International Cosmetic Ingredient Dictionary and Handbook) et les quantités en pourcentage en poids sauf mention contraire.

I. Exemples de composition

**Exemple 1 : Emulsion H/E**

| Phase huileuse (phase A) | |
|---|---|
| Huile d'abricot | 3 % |
| Huile de silicone | 5 % |
| Huile de karité | 3 % |
| Tétraoctanoate de pentaerythrityle | 3 % |
| Stéarate de glycéryl/stéarate de PEG-40 (Arlacel 165) | 3 % |
| Alcool stéarylique | 1 % |
| Filtres (Parsol MCX et Benzophénone-3) | 9,5 % |
| Phase B | |
| Amidon | 0,5 % |
| Gomme de silicone à 12 % de M.A. (DC2-9085) (soit 0,024 % de diméthiconol et 0,176 % de diméthicone) | 0,2 % |
| Gélifiants (phase C) | |
| Hydroxyéthylcellulose | 0,1 % |
| Carbomer (carbopol) | 0,6 % |
| Phase aqueuse (phase D) | |
| Conservateur | qs |
| Glycérine | 5 % |
| Eau | qsp 100 % |
| Phase E | |
| Triéthanolamine (neutralisant) | qs pH 6 |

Mode opératoire : on prépare séparément les phases huileuse et aqueuse à 70°C et on verse la phase huileuse dans la phase aqueuse sous agitation. On agite pendant 10 minutes pour faire l'émulsion, puis on ajoute la phase C puis la phase B et enfin la phase E pour neutraliser le Carbopol.

On obtient une crème apte à être appliquée sur la peau pour la protéger contre les effets de la pollution et notamment pour limiter l'adhérence des particules polluantes sur la peau.

**Exemple 2 : Emulsion H/E**

| Phase huileuse (phase A) | |
|---|---|
| Huile d'abricot | 1 % |
| Huile de silicone | 5 % |
| Octyldodecanol (Eutanol G de la société Henkel) | 1 % |
| Filtres (Parsol MCX et Benzophénone-3) | 9,5 % |

(suite)

| Phase B | |
|---|---|
| Amidon<br>Gomme de silicone à 12 % de M.A. (DC2-9085) (soit 0,024 % de diméthiconol et 0,176 % de diméthicone) | 0,5 %<br>0,2 % |
| Gélifiants (Phase C) | |
| Hydroxyéthylcellulose<br>Carbomer (carbopol) | 0,1 %<br>0,7 % |
| Phase aqueuse (phase D) | |
| Conservateur<br>Glycérine<br>Eau | qs<br>5 %<br>qsp 100 % |
| Phase E | |
| Triéthanolamine (neutralisant) | qs pH 6 |

Mode opératoire : on prépare séparément les phases huileuse et aqueuse à 70°C et on verse la phase huileuse dans la phase aqueuse sous agitation. On agite pendant 10 minutes pour faire l'émulsion, puis on ajoute la phase C, la phase B et enfin la phase E pour neutraliser le Carbopol.

On obtient une crème apte à être appliquée sur la peau pour la protéger contre les effets de la pollution et notamment pour limiter l'adhérence des particules polluantes sur la peau.

**Exemple 3 : Emulsion H/E**

| Phase huileuse (phase A) | |
|---|---|
| Huile d'abricot<br>Huile de silicone<br>Octyldodecanol (Eutanol G de la société Henkel)<br>Filtres (Parsol MCX, Mexoryl SX et Eusolex 232) | 1 %<br>5 %<br>1 %<br>9,5 % |
| Phase B | |
| Amidon<br>Gomme de silicone à 12 % de M.A. (DC2-9085) (soit 0,024 % de diméthiconol et 0,176 % de diméthicone) | 0,5 %<br>0,2 % |
| Gélifiants (Phase C) | |
| Hydroxyéthylcellulose<br>Carbomer (carbopol) | 0,1 %<br>0,7 % |
| Phase aqueuse (phase D) | |
| Conservateur<br>Glycérine<br>Eau | qs<br>5 %<br>qsp 100 % |

(suite)

| Phase E | |
|---|---|
| Triéthanolamine (neutralisant) | qs pH 6 |

Mode opératoire : on prépare séparément les phases huileuse et aqueuse à 70°C et on verse la phase huileuse dans la phase aqueuse sous agitation. On agite pendant 10 minutes pour faire l'émulsion, puis on ajoute la phase C, la phase B et enfin la phase E pour neutraliser le Carbopol.

On obtient une crème apte à être appliquée sur la peau pour la protéger contre les effets de la pollution et notamment pour limiter l'adhérence des particules polluantes sur la peau.

**Exemple 4 : Emulsion H/E**

| Phase huileuse (Phase A) | |
|---|---|
| Huile d'abricot | 1 % |
| Huile de silicone | 5 % |
| Octyldodecanol (Eutanol G de la société Henkel) | 1 % |
| Filtres (Parsol MCX, Mexoryl SX et Eusolex 232) | 9,5 % |
| Phase B | |
| Amidon | 0,5 % |
| Gélifiants (phase C) | |
| Hydroxyéthylcellulose | 0,1 % |
| Carbomer (carbopol) | 0,7 % |
| Phase aqueuse (phase D) | |
| Conservateur | qs |
| Glycérine | 5 % |
| Eau | qsp 100 % |
| Phase E | |
| Triéthanolamine (neutralisant) | qs pH 6 |

Mode opératoire : on prépare séparément les phases huileuse et aqueuse à 70°C et on verse la phase huileuse dans la phase aqueuse sous agitation. On agite pendant 10 minutes pour faire l'émulsion, puis on ajoute la phase C, puis la phase B et enfin la phase E pour neutraliser le Carbopol.

On obtient une crème apte à être appliquée sur la peau pour la protéger contre les effets de la pollution et notamment pour limiter l'adhérence des particules polluantes sur la peau.

II. Mise en évidence de l'activité anti-pollution de l'amidon seul et associé avec une gomme de silicone

Pour mettre en évidence l'activité anti-pollution de l'amidon et de l'association amidon et gomme de silicone, on a testé les compositions décrites ci-dessus contenant soit 2% d'amidon (exemple 4) soit 0,5% d'amidon et 0,2% de gomme de silicone (exemples 1 à 3). Les placebos sont les mêmes compositions sans amidon ni gomme de silicone.

1. Protocole utilisé

Matériel biologique : Epidermes humains reconstruits vendus par la société EPISKIN® (LYON, France) avec ses milieux de maintenance et d'essai (Kit).

Réactifs : (1) Particules Diesel commercialisées sous la dénomination Diesel Particules Matter 1650 par la société National Institute of Standard Technology (USA) ; (2) Air comprimé dépoussiérant sec commercialisé sous la dénomination SOUFFL'SEC par la société AF France ; (3)_sébum artificiel.

L'air comprimé sert dans le test, à éliminer les particules non attachées (adsorbées) à la surface de la peau, le but étant de mimer les turbulences d'air auxquelles la peau et les particules sont soumises dans la réalité.

Appareillage et petit matériel :

- Tubes polystyrène usage unique 10ml et bouchons ;
- Spatule inox recourbée ;
- Balance de précision au 1/10e de mg (METTLER ou similaire) ;
- Analyseur d'image avec caméra vidéo noir et blanc munie d'un objectif zoom de type Leica Q500IW ou similaire ;
- Poste de sécurité microbiologique.

2. Test : On a déposé sur l'épiderme humain reconstruit, 2 $mg/cm^2$ de sébum avec une spatule recourbée. Puis, on a laissé sécher à l'air libre (sans couvercle) sous la hotte à flux laminaire pendant deux heures.

L'application de sébum permet d'obtenir une peau plus proche de la peau humaine réelle, par exemple de la peau du visage sur laquelle il y a du sébum *in vivo*.

Ensuite, on a déposé sur l'épiderme de peau reconstruite les compositions à tester avec une spatule recourbée à raison de 2 $mg/cm^2$. On les a laissé sécher à l'air libre (sans couvercle) sous la hotte à flux laminaire pendant 30 minutes. Ensuite, on y a déposé (sous poste de sécurité) 1,5 mg de particules Diesel que l'on a réparties à la surface de l'épiderme. On a éliminé l'excès de particules par retournement de la nacelle et on a séché 10 min à l'air libre (sans couvercle), puis on a quantifié par analyse d'image les particules Diesel ayant adhérées, c'est-à-dire la surface initiale salie.

On a ensuite nettoyé (sous poste de sécurité) par pulvérisation d'air sec pendant 10 secondes à 25 cm de la nacelle. On a laissé sécher 10 minutes à l'air libre (sans couvercle), puis on a quantifié par analyse d'image les particules restantes, c'est-à-dire la surface salie restante.

3. Calculs

On calcule le pourcentage de particules éliminées selon l'équation :

$$\text{\% particules éliminées} = \frac{(\text{surface initiale salie - surface salie restante})}{\text{surface initiale salie}} \times 100$$

4. Résultats :

(Dans les tableaux ci-dessous, SD signifie « standard deviation »).

| Composés anti-pollution | % particules éliminées | SD | Significativité |
|---|---|---|---|
| Exemple 1 selon l'invention | 64,09 | 1,25 | p<0,05 (par rapport au placébo et au sébum) |
| témoin placebo | 47,09 | 3,79 | |
| témoin sébum | 36,8 | 4,8 | |

| Composés anti-pollution | % particules éliminées | SD | significativité |
|---|---|---|---|
| Exemple 2 selon l'invention | 50 | 6,1 | p<0,05 (par rapport au placébo et au sébum) |
| témoin placebo | 40,5 | 4,5 | |
| témoin sébum | 36,8 | 4,8 | |

| Composés anti-pollution | % particules éliminées | SD | significativité |
|---|---|---|---|
| Exemple 3 selon l'invention | 56,07 | 4,46 | p<0,05 (par rapport au sébum) |

(suite)

| Composés anti-pollution | % particules éliminées | SD | significativité |
|---|---|---|---|
| témoin placebo | 51,2 | 2,58 | |
| témoin sébum | 36,8 | 4,8 | |

| Composés anti-pollution | % particules éliminées | SD | Significativité |
|---|---|---|---|
| Exemple 4 selon l'invention | 58,01 | 2,96 | $p<0,05$ (par rapport au sébum) |
| témoin placebo | 46,76 | 4,54 | |
| témoin sébum | 36,8 | 4,8 | |

Les résultats indiqués dans les tableaux ci-dessus montrent un effet anti-pollution significatif (T Student, $p<0.05$) des compositions contenant un amidon ou un amidon et une gomme de silicone, vis à vis des particules standard diesel, comparativement aux épidermes surfacés avec du sébum.

## Revendications

1. Utilisation cosmétique d'au moins un amidon, comme agent cosmétique anti-pollution, dans une composition pour application topique sur les matières kératiniques.

2. Utilisation d'au moins un amidon, pour la préparation d'une composition à application topique destinée à protéger les matières kératiniques contre les effets nocifs de la pollution.

3. Utilisation selon la revendication 1 ou 2, **caractérisée par le fait que** la quantité d'amidon(s) va de 0,05 à 15 % en poids par rapport au poids total de la composition.

4. Utilisation selon l'une quelconque des revendications précédentes, **caractérisée par le fait que** l'amidon est choisi parmi les amidons de maïs, de riz, de manioc, de pomme de terre, de blé, de sorgho, de pois, natifs ou modifiés, et leurs mélanges.

5. Utilisation selon la revendication précédente, **caractérisée par le fait que** l'amidon est un amidon non modifié.

6. Utilisation selon la revendication précédente, **caractérisée par le fait que** l'amidon est un amidon de maïs.

7. Utilisation selon l'une quelconque des revendications précédentes, **caractérisée par le fait que** la composition contient en outre au moins une gomme de silicone.

8. Utilisation selon la revendication précédente, **caractérisée par le fait que** la gomme de silicone est choisie parmi les composés de formule (I) suivante :

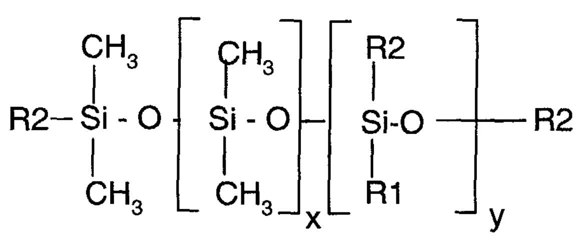

(I)

dans laquelle :

R1 représente $-CH_3$, -OH, $-C_6H_5$ ou $-OSi(CH_3)_3$,

R2 représente —$CH_3$, -OH ou -$C_6H_5$,
x = 0 ou un nombre entier et
y est un nombre entier,
y ou x + y étant des nombres entiers tels que la masse moléculaire moyenne en poids soit supérieure à 100 000, et de préférence aille de 100 000 à 1 500 000.

**9.** Utilisation selon la revendication précédente, **caractérisée en ce que** la gomme de silicone est un diméthiconol.

**10.** Utilisation selon l'une quelconque des revendications 7 à 9, **caractérisée par le fait que** la quantité de gomme (s) de silicone va de 0,01 à 10 % en poids par rapport au poids total de la composition.

**11.** Utilisation selon l'une quelconque des revendications précédentes, **caractérisée par le fait que** la composition contient en outre un filtre solaire.

**12.** Composition à application topique, contenant dans un milieu physiologiquement acceptable, au moins un amidon non modifié, choisi parmi les amidons de maïs, de riz, de manioc, de pomme de terre, de blé, de sorgho et de pois, et au moins une gomme de silicone.

**13.** Composition selon la revendication précédente, **caractérisée par le fait que** l'amidon est un amidon de maïs.

**14.** Composition selon la revendication 12 ou 13, **caractérisée par le fait que** la gomme de silicone est choisie parmi les composés de formule (I) suivante :

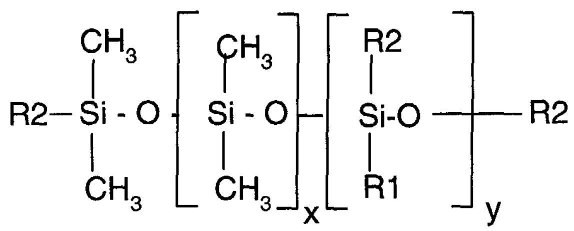
(I)

dans laquelle :

R1 représente —$CH_3$, -OH, -$C_6H_5$ ou -$OSi(CH_3)_3$,
R2 représente —$CH_3$, -OH ou -$C_6H_5$,
x = 0 ou un nombre entier et
y est un nombre entier,
y ou x + y étant des nombres entiers tels que la masse moléculaire moyenne en poids soit supérieure à 100 000, et de préférence aille de 100 000 et 1 500 000.

**15.** Composition selon la revendication précédente, **caractérisée en ce que** la gomme de silicone est un diméthiconol.

**16.** Composition selon l'une quelconque des revendications 12 à 15, **caractérisée par le fait que** la quantité d'amidon (s) va de 0,05 à 15 % en poids par rapport au poids total de la composition, et de préférence de 0,1 à 10 % en poids par rapport au poids total de la composition.

**17.** Composition selon l'une quelconque des revendications 12 à 16, **caractérisée en ce que** la quantité de gomme (s) de silicone va de 0,01 à 10 % en poids par rapport au poids total de la composition, et de préférence de 0,05 à 5 % en poids par rapport au poids total de la composition.

**18.** Composition selon l'une quelconque des revendications 12 à 17, **caractérisée par le fait qu'**elle contient en outre au moins un filtre solaire.

**19.** Composition selon la revendication précédente, **caractérisée par le fait que** la quantité de filtre(s) va de 0,5 à 20 % en poids par rapport au poids total de la composition.

**20.** Composition selon la revendication 18 ou 19, **caractérisée par le fait que** le filtre est choisi parmi l'octylméthoxycinnamate, l'acide téréphtalylidène di-camphre sulfonique, le benzophénone-3, l'acide phénylbenzimidazole sulfonique et leurs mélanges.

**21.** Composition selon l'une quelconque des revendications 12 à 20 **caractérisée par le fait qu'**elle se présente sous forme d'une émulsion.

**22.** Procédé de traitement cosmétique en vue de protéger une matière kératinique contre les effets de la pollution, consistant à appliquer sur la matière kératinique, une composition contenant dans un milieu physiologiquement acceptable, une quantité efficace d'au moins un amidon.

**23.** Procédé de traitement cosmétique d'une matière kératinique en vue d'améliorer sa respiration cellulaire et/ou de diminuer sa desquamation et/ou pour éviter de la ternir et/ou de la salir, consistant à appliquer sur la matière kératinique, une composition contenant dans un milieu physiologiquement acceptable, une quantité efficace d'au moins un amidon.

**24.** Procédé selon la revendication 22 ou 23, **caractérisé par le fait que** la composition contient en outre au moins une gomme de silicone.

**25.** Procédé selon l'une quelconque des revendications 22 à 24, **caractérisé par le fait que** la matière kératinique est la peau.

Office européen des brevets

# RAPPORT DE RECHERCHE EUROPEENNE

Numéro de la demande
EP 02 29 0093

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | DE 39 38 284 A (J. KLOSA) 22 mars 1990 (1990-03-22) * colonne 1, ligne 1-10; exemples 2,5 * | 1 | A61K7/48 A61K7/40 |
| X | EP 1 022 017 A (STADA ARZNEIMITTEL AG) 26 juillet 2000 (2000-07-26) * exemples 7,16 * | 12 | |
| X | EP 0 418 443 A (NEUTROGENA CORP.) 27 mars 1991 (1991-03-27) * le document en entier * | 12 | |
| X | WO 96 22073 A (NATIONAL STARCH AND CHEM. INV. HOLD. CO.) 25 juillet 1996 (1996-07-25) * exemples 27,31 * | 12 | |
| X | EP 1 051 967 A (GRÄFE CHEMIE GMBH) 15 novembre 2000 (2000-11-15) * page 7, ligne 23-59; revendication 1 * | 12 | DOMAINES TECHNIQUES RECHERCHES (Int.Cl.7) |
| X | US 5 945 447 A (H. FALLICK) 31 août 1999 (1999-08-31) * le document en entier * | 12 | A61K |
| X | WO 00 35413 A (THE PROCTER & GAMBLE CO.) 22 juin 2000 (2000-06-22) * exemple 1A * | 12 | |
| P,X | US 6 277 893 B1 (T. BABENKO) 21 août 2001 (2001-08-21) * le document en entier * | 12 | |

Le présent rapport a été établi pour toutes les revendications

| Lieu de la recherche | Date d'achèvement de la recherche | Examinateur |
|---|---|---|
| LA HAYE | 8 mai 2002 | Glikman, J-F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 02 29 0093

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-05-2002

| Document brevet cité au rapport de recherche | | Date de publication | Membre(s) de la famille de brevet(s) | | | Date de publication |
|---|---|---|---|---|---|---|
| DE 3938284 | A | 22-03-1990 | DE | 3938284 | A1 | 22-03-1990 |
| EP 1022017 | A | 26-07-2000 | DE | 29900938 | U1 | 06-05-1999 |
| | | | EP | 1022017 | A2 | 26-07-2000 |
| EP 418443 | A | 27-03-1991 | CA | 1330309 | A1 | 21-06-1994 |
| | | | JP | 3112921 | A | 14-05-1991 |
| | | | US | 4894222 | A | 16-01-1990 |
| | | | AU | 625368 | B2 | 09-07-1992 |
| | | | AU | 4152189 | A | 20-06-1991 |
| | | | DE | 68914455 | D1 | 11-05-1994 |
| | | | DE | 68914455 | T2 | 25-08-1994 |
| | | | EP | 0418443 | A1 | 27-03-1991 |
| WO 9622073 | A | 25-07-1996 | AU | 4701096 | A | 07-08-1996 |
| | | | CA | 2210160 | A1 | 25-07-1996 |
| | | | EP | 0804140 | A2 | 05-11-1997 |
| | | | JP | 10506921 | T | 07-07-1998 |
| | | | WO | 9622073 | A2 | 25-07-1996 |
| | | | US | 5871756 | A | 16-02-1999 |
| EP 1051967 | A | 15-11-2000 | DE | 19921707 | A1 | 16-11-2000 |
| | | | EP | 1051967 | A2 | 15-11-2000 |
| US 5945447 | A | 31-08-1999 | US | 5846996 | A | 08-12-1998 |
| WO 0035413 | A | 22-06-2000 | AU | 2170200 | A | 03-07-2000 |
| | | | WO | 0035413 | A1 | 22-06-2000 |
| US 6277893 | B1 | 21-08-2001 | AUCUN | | | |

EPO FORM P0460

Pour tout renseignement concernant cette annexe : voir Journal Officiel de l'Office européen des brevets, No.12/82